# EUROPÄISCHE PATENTANMELDUNG

(11) **EP 4 778 498 A2**
(43) Veröffentlichungstag der Anmeldung: **22.07.2026**
(21) Anmeldenummer: 26183048.3
(22) Anmeldetag: 29.09.2022
(51) Int. Cl.: A61F 2/66

(54) **ORTHOPÄDIETECHNISCHES SYSTEM UND PROTHESENFUSS MIT EINEM SOLCHEN**

(30) Priorität: 30.09.2021 DE 102021125385
(62) Teilanmeldung aus: 24210770.4
(71) Anmelder: Ottobock SE & Co. KGaA, 37115 Duderstadt (DE)
(72) Erfinder: PUSCH, Martin, 37115 Duderstadt (DE); KRENZ, Hannes, 37115 Duderstadt (DE); GEHRMANN, Georg, 37115 Duderstadt (DE); MOENICKE, Carsten, 37115 Duderstadt (DE); BENSTEM, Nils, 37115 Duderstadt (DE)
(74) Vertreter: Gramm, Lins & Partner Patent- und Rechtsanwälte PartGmbB

(57) **Zusammenfassung**

Die Erfindung betrifft ein orthopädietechnisches System mit einem Blattfederelement (40) und einer auf der Hauptfläche des Blattfederelements (40) abgestützten Lagerungskomponente (600), die an dem Blattfederelement (40) festgelegt ist, wobei zwischen der Lagerungskomponente (600) und der Hauptfläche (41) des Blattfederelementes (40) zumindest ein Elastomerelement (71, 72) angeordnet ist, das Zugkräfte und Scherkräfte übertragend an dem Blattfederelement (40) und der Lagerungskomponente (600) befestigt ist, wobei an der Lagerungskomponente (600) zumindest ein Anschlag (630) angeordnet ist, der in einer Ausgangsstellung beabstandet zu einer Nebenfläche (43) und/oder einem Anschlagelement des Blattfederelementes (40) angeordnet ist.

## Beschreibung

Die Erfindung betrifft ein orthopädietechnisches System mit einem Blattfederelement und einer auf einer Hauptfläche des Blattfederelementes abgestützten Lagerungskomponente, die an dem Blattfederelement festgelegt ist sowie einen Prothesenfuß mit einem solchen orthopädietechnischen System.

Blattfederelemente werden häufig in orthopädietechnischen Systemen eingesetzt, um Verformungsenergien zu speichern und gleichzeitig Kraftbegrenzungen zu verwirklichen. Blattfederelemente der modernen Bauart bestehen aus Faserverbundwerkstoffen oder weisen Komponenten aus Faserverbundwerkstoffen auf und sind Teil komplexer Systeme, beispielsweise von Prothesenfüßen. Um die Blattfederelemente effektiv einzusetzen, müssen diese in Halterungen eingebaut werden, damit weitere Komponenten an dem Blattfederelement gelagert werden können. Die Befestigung von Halterungen oder anderen Bauteilen an einem Blattfederelement erfolgt beispielsweise formschlüssig, indem das zum montierende Bauteil, beispielsweise ein Metallbauteil, an dem Blattfederelement angeschraubt wird. Eine solche Lösung ist in der US 10 390 974 B2 und der EP 3 128 958 B1 beschrieben.

Aus der WO 2020/1523 41 A1 ist bekannt, dass ein Blattfederelement über eine Klebeverbindung, eine Klemmverbindung und/oder eine Formschlussverbindung mit einem Träger verbunden sein kann. Bei Formschlussverbindungen über Schrauben werden Bohrungen in das Blattfederelement eingebracht, durch die dann entsprechende Bolzen oder Schrauben hindurchgeführt und somit der weiteren Komponente verbunden werden. Bohrungen schwächen die Blattfederkomponente und sind daher nicht überall anzubringen, insbesondere Faserverbundwerkstoffe werden in ihren mechanischen Eigenschaften durch Bohrungen oder Störungen in der Faserstruktur beeinträchtigt. Insbesondere in Bereichen großer Deformationen der Blattfederelemente lassen sich Anbauteile nicht oder nur schwer montieren.

Aufgabe der vorliegenden Erfindung ist es daher ein orthopädietechnisches System mit einem Blattfederelement bereitzustellen, das eine größere Gestaltungsfreiheit ohne Einschränkung der Haltbarkeit und Belastbarkeit des Gesamtsystems ermöglicht.

Erfindungsgemäß wird diese Aufgabe durch ein orthopädietechnisches System mit den Merkmalen des Hauptanspruches und einen Prothesenfuß mit einem solchen orthopädietechnischen System gelöst. Vorteilhafte Ausgestaltungen und Weiterbildungen der Erfindung sind in der Beschreibung, den Unteransprüchen sowie den Figuren offenbart.

Das orthopädietechnische System mit einem Blattfederelement und einer auf einer Hauptfläche des Blattfederelementes abgestützten Lagerungskomponente, die an dem Blattfederelement festgelegt ist, sieht vor, dass zwischen der Lagerungskomponente und der Hauptfläche des Blattfederelementes zumindest ein Elastomerelement angeordnet ist, das Zugkräfte und Scherkräfte übertragend an dem Blattfederelement und der Lagerungskomponente befestigt ist. Zwischen der Lagerungskomponente und dem Blattfederelement ist somit eine elastische Anbindung vorhanden, die eine Relativbewegung zwischen dem Blattfederelement und der Lagerungskomponente ermöglicht. Dadurch ist es möglich, trotz einer sicheren und belastbaren Zuordnung der Lagerungskomponente an dem Blattfederelement auch in Bereichen hoher Verformungen des Blattfederelementes eine Anordnung und Positionierung der Lagerungskomponente zu erreichen. Die Befestigung der Lagerungskomponente an dem Blattfederelement in der Art und Weise, dass auch Zugkräfte und Scherkräfte, die über die Lagerungskomponente auf das Blattfederelement aufgebracht werden, sicher und dauerhaft auf das Blattfederelement übertragen werden können, ermöglicht eine nahezu beliebige Positionierung der Lagerungskomponente auf dem Blattfederelement, ungeachtet der Verformungssituation des Blattfederelementes bei einer Benutzung des orthopädietechnischen Systems. Dadurch erhöht sich der Gestaltungsfreiraum bei der Konstruktion des orthopädietechnischen Systems oder des Gesamtsystems, von dem das orthopädietechnische System ein Teil ist.

In einer Ausgestaltung weist die Lagerungskomponente zumindest eine ebene Abstützfläche auf, über die sich das Elastomerelement auf der Lagerungskomponente abstützt und darüber die Verbindung zu dem Blattfederelement herstellt. Die ebene Abstützfläche ermöglicht eine großflächige Einleitung von Kräften und vermeidet Belastungsspitzen von Kräften, die von der in der Regel starren Lagerungskomponente über das Elastomerelement in das Blattfederelement eingeleitet werden.

In einer Weiterbildung ist die Lagerungskomponente mit mehreren, voneinander beabstandeten Abstützflächen versehen, sodass eine Lagerung und Befestigung der Lagerungskomponente an mehreren, voneinander beabstandeten Bereichen des Blattfederelementes erfolgt. Die beiden Abstützflächen werden brückenartig durch die Lagerungskomponente miteinander verbunden und ermöglichen eine optimierte Einleitung von Kräften und eine verbesserte Lastverteilung, sodass die mechanischen Eigenschaften des Blattfederelementes optimal ausgenutzt werden können.

Das Elastomerelement ist in einer Ausgestaltung über eine Vorspanneinrichtung an dem Blattfederelement befestigt. Die Vorspanneinrichtung kann beispielsweise als eine Klammer, ein Klemmsystem, ein Gurt oder eine Verschraubung ausgebildet sein. Durch die Vorspannung über die Vorspanneinrichtung werden die drei Komponenten des Systems, das Blattfederelement, die Lagerungskomponente und das Elastomerelement, aneinander gepresst, wodurch verhindert wird, dass die Zuordnung der Komponenten zueinander aufgelöst wird. Eine Geräuschentwicklung durch Aufeinanderschlagen von Bauteilen wird vermieden, darüber hinaus wird dem Nutzer des orthopädietechnischen Systems ein sicheres Gefühl durch eine stabile Krafteinleitung vermittelt.

Das zumindest eine Elastomerelement ist in einer Ausgestaltung mit der Lagerungskomponente und/oder dem Blattfederelement verklebt. Bei einer Verklebung des Elastomerelementes sowohl mit der Lagerungskomponente als auch mit dem Blattfederelement wird eine stoffschlüssige Kopplung aller drei Elemente des Systems auf eine einfache und preiswerte sowie dauerhafte Art und Weise gewährleistet. Eine Beeinträchtigung der Struktur des Blattfederelementes erfolgt durch die Befestigung der Lagerungskomponente nicht. Gleiches gilt für eine Verklebung des Elastomerelementes mit dem Blattfederelement und einer Kopplung und Befestigung des Elastomerelementes mit der Lagerungskomponente über eine andere Befestigungseinrichtung oder ein Befestigungselement, beispielsweise durch Schrauben. Es besteht auch die Möglichkeit, durch Klemmen über Klemmeinrichtungen eine Befestigung des Blattfederelementes an der Lagerungskomponente unter Zwischenschaltung des Elastomerelementes zu bewirken. Auch wenn eine Verschraubung mit einem Durchgangsloch durch das Blattfederelement erfolgt, wird über die Zwischenschaltung des Elastomerelementes die mechanische Belastung einer Kraftübertragung über die Lagerungskomponente verringert und ein Vorteil hinsichtlich der Gestaltungsfreiheit und Haltbarkeit des orthopädietechnischen Systems erreicht.

Die Lagerungskomponente kann zumindest einen Anschlag aufweisen, der in einer Ausgangsstellung, in der keine Kräfte durch die Benutzung des orthopädietechnischen Systems von der Lagerungskomponente auf das Blattfederelement übertragen werden, beanstandet zu einer Nebenfläche oder einem Anschlagelement des Blattfederelementes angeordnet ist. Blattfederelemente weisen eine Längserstreckung auf und sind im Querschnitt zu der Längserstreckung im Wesentlichen rechteckig mit zwei langen Seiten und zwei einander gegenüberliegenden Kurzseiten ausgebildet. Die Oberfläche, die den langen Seiten zugeordnet ist, ist eine Hauptfläche, die Oberfläche, die den kurzen Seiten oder Schmalseiten zugeordnet ist, ist eine Nebenfläche. Ein Anschlag oder mehrere Anschläge können seitlich neben dem Blattfederelement angeordnet sein. Durch den Abstand zu der Nebenfläche ist eine Beweglichkeit quer zu der Längserstreckung des Blattfederelementes möglich. Ausgleichsbewegungen und Verschiebebewegungen werden über das Elastomerelement ermöglicht und aufgefangen. Die Verformungen des Elastomerelementes werden bis zu einem Kontakt des Anschlages mit dem Blattfederelement zugelassen. Eine Nebenfläche kann auch innerhalb des Blattfederelementes in einer Bohrung oder in einem Schlitz ausgebildet sein. So kann ein an oder in der Lagerungskomponente angeordneter Anschlag in einen Schlitz, eine Bohrung oder eine andere Ausnehmung eingreifen, wobei ohne eine Belastung, insbesondere ohne eine Belastung in der Hauptebene, der Anschlag nicht in unmittelbaren Kontakt mit dem Blattfederelement steht. Alternativ oder ergänzend zu einem direkten Kontakt mit dem Blattfederelement kann an dem Blattfederelement ein Anschlagselement angeordnet oder ausgebildet sein, das gegen einen Anschlag oder eine Ausnehmung der Lagerungskomponente anschlägt oder in Kontakt damit tritt, wenn eine Belastungsgrenze beschritten wird und die Relativverlagerung von Lagerungskomponente zu Blattfederelement zu groß ist. Die Verlagerung kann eine Verdrehung oder eine Verschiebung der Lagerungskomponente zu dem Blattfederelement sein.

In einer Ausgestaltung sind mehrere Anschläge oder Anschlagelemente einander gegenüberliegend angeordnet oder ausgebildet, insbesondere sind mehrere Anschläge seitlich neben dem Blattfederelement und/oder dem Anschlagelement angeordnet und rahmen dieses oder diese von zwei oder mehr Seiten ein. Ein Anschlag erfolgt dann bevorzugt an einer der Nebenflächen oder an zwei Anschlagelementen an dem Blattfederelement.

Vorteilhafterweise ist die Lagerungskomponente in drei rotatorischen und drei translatorischen Freiheitsgraden elastisch auf dem Blattfederelement gelagert, wobei aufgrund der Abmessungen des Elastomerelementes jeweils nur geringe Verdrehungen und/oder Verschiebungen in die jeweiligen Richtungen bzw. um die jeweiligen Achsen möglich sind. In einer vorteilhaften Ausgestaltung ist vorgesehen, dass eine Torsion in der Transversalebene in einem Bereich zwischen +/- 1° und +/-5°, insbesondere +/- 2,5° beträgt. Aufgrund des Umstandes, dass keine starre Befestigung des Blattfederelementes an der Lagerungskomponente vorhanden ist, wird eine Nachgiebigkeit bei Torsionsbelastungen insbesondere in der Frontalebene und der Transversalebene ermöglicht, wobei die Bezugsebene hierfür die Ebene ist, in der die Hauptfläche des Blattfederelementes liegt. Die Verbindung zwischen der Lagerungskomponente und dem Blattfederelement wird dadurch so ausgelegt, dass eine Relativbewegung zwischen den beiden Bauteilen in einem begrenzten Ausmaß möglich ist. Gleichzeitig wird eine starke Verformung des Blattfederelementes in allen Richtungen in einem begrenzten Umfang möglich, ohne dass es zu einer direkten Kollision zwischen dem Blattfederelement und der Lagerungskomponente kommt. Dennoch werden hohe mechanische Kräfte und Belastungen von der Lagerungskomponente auf das Blattfederelement übertragen, wobei die Übertragung von Normalkräften von der Blattfeder auf den Lagerbock die hauptsächliche Kraftübertragungseinrichtung darstellen wird.

Vorteilhafterweise ist das Blattfederelement aus einem Faserverbundwerkstoff hergestellt, die Lagerungskomponente ist insbesondere aus einem Metall oder einer Metalllegierung, insbesondere einer Leichtmetalllegierung hergestellt. Damit werden die jeweiligen mechanischen Belastungen durch das jeweilige Material optimal aufgenommen und weitergeleitet.

Die Lagerungskomponente ist insbesondere als ein Lagerbock mit einer Lageraufnahme ausgebildet, sodass eine gelenkige Anbindung weiterer Komponenten eines orthopädietechnischen Gesamtsystems, insbesondere einer weiteren Prothesenkomponente, erfolgen kann. Statt einer gelenkigen Anbindung weiterer Komponenten an die Lagerungskomponente kann eine oder können mehrere Komponenten starr oder federnd an der Lagerungskomponente befestigt sein. Ein Pyramidenadapter kann direkt an der Lagerungskomponente befestigt oder ausgebildet sein oder über ein elastisches Bauteil mit der übrigen Lagerungskomponente gekoppelt sein. Die Verbindung anderer Bauteile mit der Lagerungskomponente, insbesondere eine seitliche und/oder proximale Anbindung, kann auch drehfest oder drehstarr ausgeführt sein, worunter sowohl eine einstückige Ausgestaltung als auch eine mehrteilige Ausgestaltung zu verstehen ist.

Die Lagerungskomponente ist in einer Ausgestaltung in dem mittleren Drittel zwischen den Enden des Blattfederelementes angeordnet, wodurch sich eine Krafteinleitung in denjenigen Bereichen des Blattfederelementes verwirklichen lässt, die besonders nachgiebig ausgebildet sind, wenn die übrige Lagerung des Blattfederelementes an dessen Enden stattfindet. Die Krafteinleitung in Bereichen mit hoher Deformation lässt es zu, dass die Federeigenschaften des Blattfederelementes gut ausgenutzt werden und ein feines Ansprechen bei Einleitung von Kräften von der Lagerungskomponente über das Elastomerelement in das Blattfederelement gegeben ist.

Das Elastomerelement oder die Elastomerelemente bestehen bevorzugt aus einem dauerelastischen Material, insbesondere aus einem Polyurethan-Elastomer, das vorteilhafterweise eine Shore-Härte A zwischen A40 und A80, bevorzugt zwischen A50 und A70 und besonders bevorzugt zwischen A55 und A65 aufweist. Die Verwendung anderer, über die Einsatzdauer dauerhaft elastischer und dauerfester Elastomere ist ebenfalls möglich.

Die Erfindung betrifft insbesondere einen Prothesenfußeinsatz mit einem orthopädietechnischen System, wie es oben beschrieben worden ist. Mit einer solchen Ausgestaltung ist es möglich, die Reduktion der Belastung des Blattfederelementes aufgrund einer großflächigen Lasteinleitung über die gesamte Breite der Hauptseite zu ermöglichen. Belastungsspitzen durch eine direkte Kopplung von der Lagerungskomponente auf dem Blattfederelement werden vermieden. Darüber hinaus stellt sich eine erhöhte Flexibilität des Prothesenfußes für den Prothesennutzer ein, da sich eine rotatorische Nachgiebigkeit des Prothesenfußes in der Frontalebene über die Anbindung der Lagerungskomponente unter Zwischenschaltung zumindest eines Elastomerelementes ergibt. Auch die Nutzung des Prothesenfußes bei Drehungen wird erleichtert, da eine rotatorische Nachgiebigkeit in der Transversalebene vorhanden ist.

Nachfolgend werden Ausführungsbeispiele der Erfindung anhand der Figuren näher erläutert. Gleiche Bezugszeichen bezeichnen gleiche Komponenten. Es zeigen:
Figur 1 - ein orthopädietechnisches System in Explosionsdarstellung;
Figur 2 - schematische Schnittdarstellungen eines Prothesenfußes;
Figur 3 - eine schematische Gesamtdarstellung eines Prothesenfußes;
Figur 4 - eine Untenansicht auf ein orthopädietechnisches System;
Figur 5 - eine Frontalansicht auf ein orthopädietechnisches System;
Figur 6 - eine Untenansicht auf eine Lagerungskomponente;
Figur 7 - eine vergrößerte Schnittdarstellung durch eine Variante;
Figur 8 - eine Querschnittsansicht durch die Variante gemäß Figur 7; sowie
Figur 9 - drei Ansichten einer geklemmten Variante.

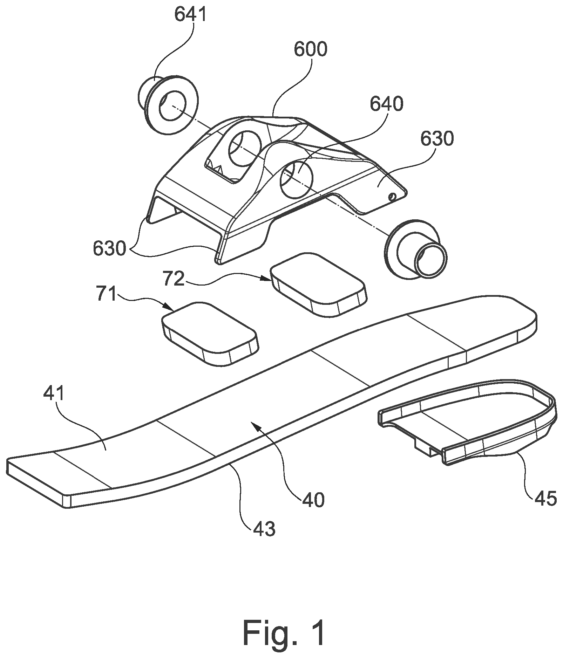

In der Figur 1 ist in einer Explosionsdarstellung ein orthopädietechnisches System mit einer Lagerungskomponente 600 in Gestalt eines Lagerbockes dargestellt, das eine Lageraufnahme 640 mit dazugehörigen Lagerschalen 641 aufweist. In der Lageraufnahme 640 ist eine Achse eingeführt, um die herum die Lagerungskomponente 600 verschwenkt werden kann. Die Lagerungskomponente 600 kann beispielsweise an einem Träger schwenkbar befestigt sein, der wiederum eine proximale Befestigungseinrichtung zur Festlegung an einer weiteren Prothesenkomponente aufweist, beispielsweise als Teil eines Prothesenfußes, der an einem Knöchelgelenk oder an einem Unterschenkelrohr oder einem Unterschenkelschaft befestigt wird. Die Lagerungskomponente 600 ist brückenartig aufgebaut und weist auf der Unterseite zwei ebene Abstützflächen auf, die später näher erläutert werden.

Unterhalb der Lagerungskomponente 600 ist ein Blattfederelement 40 aus einem Faserverbundwerkstoff dargestellt. Das Blattfederelement 40 weist einen im Wesentlichen rechteckigen Querschnitt auf, mit einer Oberseite und einer Unterseite als Hauptfläche 41 und zwei kurzen Seitenkanten als Nebenflächen 43. Das Blattfederelement 40 ist im Wesentlichen geradlinig ausgebildet, kann aber auch eine leichte Krümmung oder eine wellenartige Formgebung aufweisen. An der Unterseite des Blattfederelementes 40 ist ein Fersenelement 45 festlegbar. Alternativ zu dem Fersenelement 45 können Polsterelemente oder Kopplungseinrichtungen zur Anbindung weiterer Komponenten eines Gesamtsystems an der Unterseite und/oder Oberseite des Blattfederelementes 40 befestigt sein.

Zwischen der Oberseite auf der Hauptfläche 41 des Blattfederelementes 40 und der Unterseite mit den Abstützflächen der Lagerungskomponente 600 sind in dem dargestellten Ausführungsbeispiel zwei Elastomerelemente 71, 72 angeordnet, die flächig sowohl auf dem Blattfederelement 40 als auch an der Lagerungskomponente 600 anliegen. Seitlich neben dem Blattfederelement 40 und neben den Elastomerelementen 71, 72 befinden sich Anschläge 630, die an der Lagerungskomponente 600 ausgebildet sind. Die Anschläge 630 sind nach unten ragende Vorsprünge, die eine seitliche Begrenzung einer Verlagerung der Lagerungskomponente 600 relativ zu dem Blattfederelement 40 gewährleisten. Die Elastomerelemente 71, 72 sind beanstandet zueinander an dem Blattfederelement 40 und der Lagerungskomponente 600 befestigt, insbesondere verklebt oder verschweißt. Alternative Befestigungsarten, beispielsweise eine formschlüssige Befestigung zusätzlich oder alternativ dazu, sind vorgesehen, sofern sie eine Übertragung von Scherkräften und Zugkräften auf die jeweiligen Elastomerelemente 71, 72 ermöglichen.

Alternativ zu den insgesamt vier Anschlägen 630 können auch nur zwei diagonal, gegenüberliegende oder einseitig hintereinander angeordnete Anschläge 630 angeordnet oder ausgebildet sein. Eine weitere Alternative besteht darin, dass statt einer seitlichen Anordnung und einem möglichen Anschlag der Anschläge 630 an den Nebenflächen 43 ein Anschlagelement auf der Oberfläche des Blattfederelementes 40 angeordnet oder ausgebildet ist, das von zwei oder mehr Anschlägen eingerahmt wird und eine Relativverlagerung des Blattfederelementes 40 relativ zu der Lagerungskomponente 600 zunächst zulässt und dann begrenzt, wenn eine Grenzbelastung vorliegt. Dadurch wird eine zu große Deformation der Elastomerelemente 71, 72 begrenzt und die maximale Verlagerung von der Lagerungskomponente 600 zu dem Blattfederelement 40 festgelegt. Eine solche Bewegungseinschränkung kann auch dadurch erfolgen, dass in dem Blattfederelement 40 ein Schlitz, eine Ausnehmung oder eine Einbuchtung ausgebildet oder eingebracht ist, in den oder die ein entsprechendes Element, beispielsweise ein Zapfen, eine Lasche oder ein Stift eingeführt ist und nach Überwindung elastischer Rückstellkräfte der Elastomerelemente 71, 72 in Anschlag mit dem Blattfederelement 40 tritt.

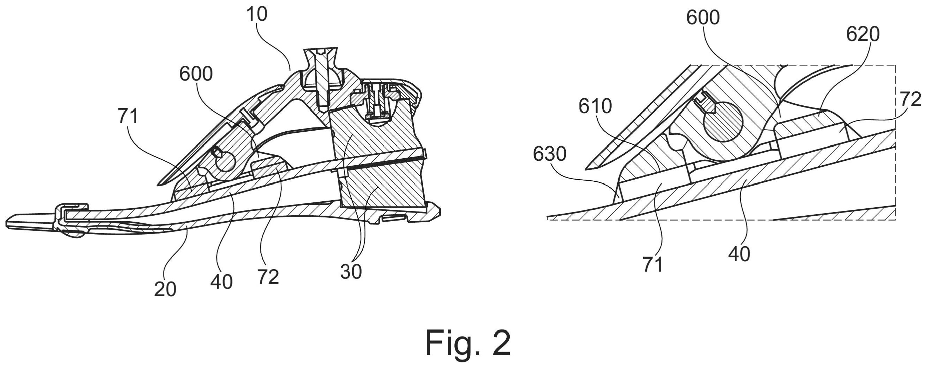

In der Figur 2 sind Schnittdarstellungen durch einen Prothesenfuß mit einem oben beschriebenen orthopädietechnischen System gezeigt. Die oben beschriebene Lagerungskomponente 600 ist ebenso zu erkennen wie die beiden Elastomerelemente 71, 72 und das Blattfederelement 40, auf dessen Oberseite die Lagerungskomponente 600 mit den Anschlägen 630 angeordnet ist. An der Unterseite der Lagerungskomponente 600 sind die jeweiligen ebenen Abstützflächen 610, 620 ausgebildet, die im Wesentlichen parallel zu der Hauptfläche 41 des Blattfederelementes 40 verlaufen. In dem dargestellten Ausführungsbeispiel sind die Elastomerelemente 71, 72 sowohl mit dem Blattfederelement 40 als auch mit der Lagerungskomponente 600 verklebt. In dem Fersenbereich des Prothesenfußes sind an der Oberseite und Unterseite des Blattfederelementes 40 Polsterelemente 30 angeordnet und befestigt. Das untere Polsterelement 30 stützt sich auf einer Basisfeder 20 ab, die in dem Vorderfußbereich mit dem Blattfederelement 40 gekoppelt oder verbunden ist. Das obere Polsterelement 30 stützt sich an einem Träger 10 ab, an dem wiederum die Lagerungskomponente 600 verschwenkbar um die Schwenkachse in der Lageraufnahme 640 gelagert ist. An der Oberseite des Trägers 10 ist ein Pyramidenadapter zur Befestigung des Prothesenfußes an einer proximalen Prothesenkomponente befestigt.

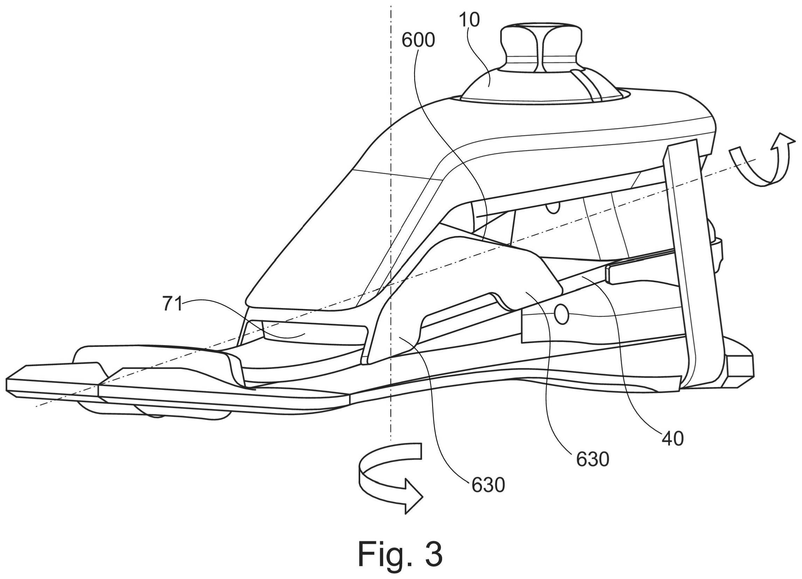

In der Figur 3 ist der Prothesenfuß gemäß Figur 2 in einer perspektivischen Gesamtdarstellung gezeigt. Der Träger 10 ist über eine Spanneinrichtung gegenüber der Basisfeder 20 vorgespannt und klemmt das Blattfederelement 40 zwischen den beiden Polsterelementen 30 ein. Das obere Polsterelement 30 ermöglicht eine geringfügige Verlagerung des Trägers 10 relativ zu dem Blattfederelement 40 in dem Fersenbereich oder in dem hinteren Bereich. Die elastische Lagerung und die Anordnung der Anschläge 630 beanstandet zu den Nebenflächen 43 des Blattfederelementes 40 erlauben eine kontrollierte, geringfügige Verdrehung sowohl um die Achse senkrecht zu der Transversalebene als auch um eine Achse senkrecht zu der Frontalebene. Grundsätzlich ist auch eine Verdrehung um eine Achse senkrecht zu der Sagittalebene möglich, die im Wesentlichen parallel zu der Achse der Lageraufnahme 640 verläuft. Aufgrund der schwenkbaren Lagerung des Trägers 10 an der Lagerungskomponente wird sich aber eine gleichmäßige Belastung sowohl auf dem hinteren als auch auf dem vorderen Elastomerelement 71, 72 einstellen, sodass eine Rotation um diesen Freiheitsgrad zwar möglich, praktisch jedoch nicht relevant ist. Neben einer Verdrehung um drei rotatorische Freiheitsgrade erlaubt die Lagerung über die Elastomerelemente 71, 72 auch eine Verlagerung in drei translatorischen Freiheitsgraden.

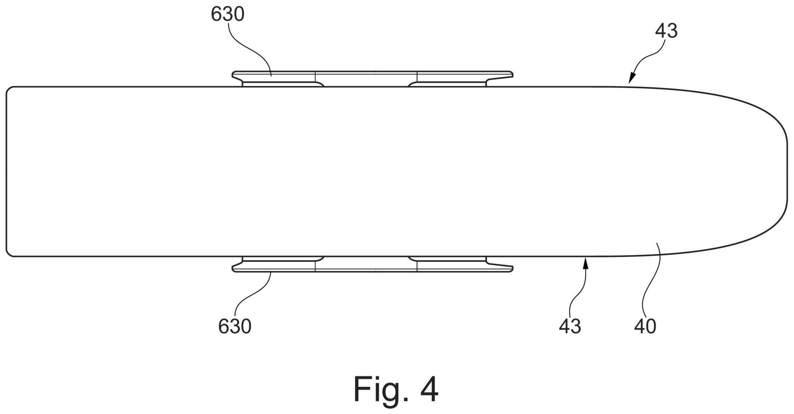

In der Untenansicht der Figur 4 ist zu erkennen, dass die Nebenflächen 43 des Federelementes 40 beanstandet zu den Anschlägen 630 der Lageraufnahme 600 angeordnet sind und damit eine Verdrehung und eine Verschiebung des gesamten Federelementes 40 relativ zu der Lagerungskomponente 600 in allen drei rotatorischen und drei translatorischen Freiheitsgraden möglich ist.

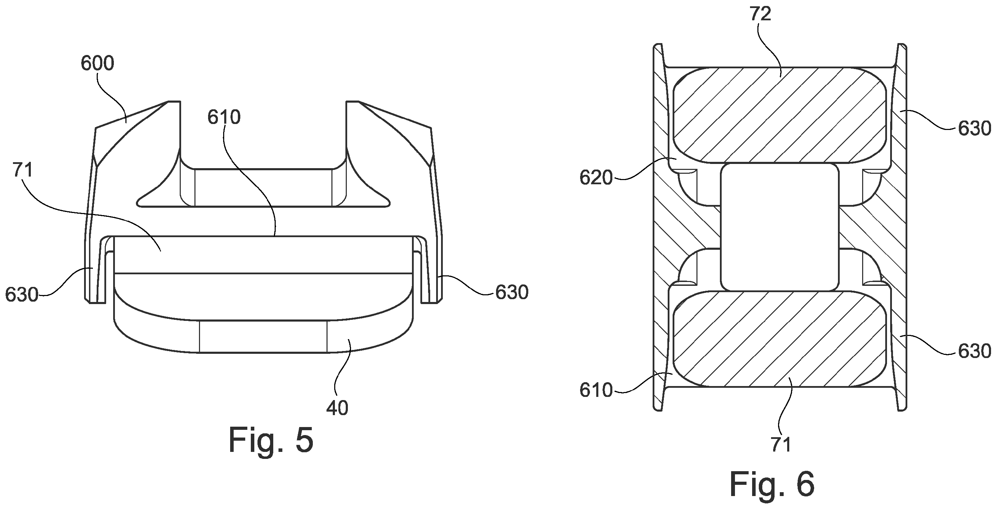

In der Figur 5 ist eine Frontalansicht der Lagerungskomponente 600 mit der vorderen, ebenen Anlagefläche 610, den beiden seitlichen Anschlägen 630 sowie den Elastomerelementen 71, 72 zwischen dem Blattfederelement 40 und der Anlagefläche 610 zu erkennen. In der Figur 6 ist in einer Untenansicht die Lagerungskomponente 600 mit den beiden ebenen Abstützflächen 610, 620 für die beiden beanstandet zueinander angeordneten Elastomerelemente 71, 72 zu erkennen. Die Anschläge 630 sind jeweils rechts und links oder medial und lateral zu den seitlichen Kanten der Elastomerelemente 71, 72 in einem Abstand dazu positioniert. Die Elastomerelemente 71, 72 erstrecken sich über die gesamte Breite des Blattfederelementes 40 und ermöglichen so eine vollflächige, aufgrund der elastischen Eigenschaften gleichmäßige Krafteinleitung auf das Blattfederelement 40 über dessen gesamte Breite. Aufgrund der großflächigen Einleitung von Kräften werden Belastungsspitzen auf das Blattfederelement 40 vermieden, die mechanische Belastung verringert sowie eine Beschädigung verhindert. Die Lagerungskomponente 600 ist brückenartig aufgebaut, wodurch zwischen den beiden Elastomerelementen 71, 72 ein Freiraum oder eine Lücke entsteht, sodass bei einer zentralen Belastung über die Lageraufnahme 640 die Krafteinleitung entlang der Längserstreckung des Blattfederelementes 40 auf zwei beanstandete Bereiche oder Flächen aufgeteilt wird.

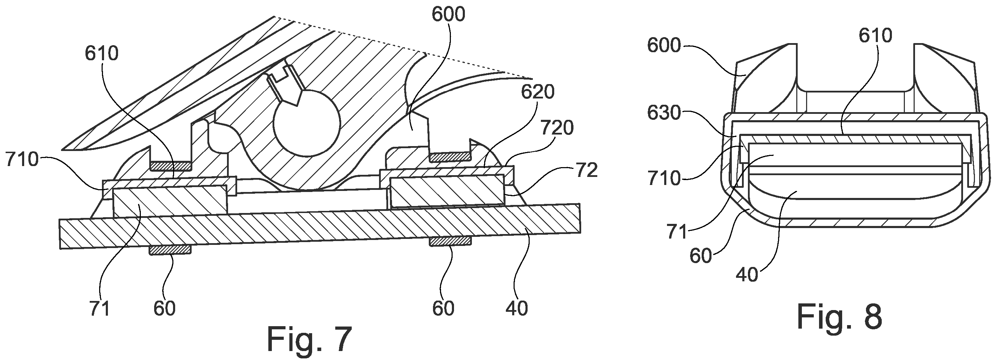

In den Figuren 7 und 8 sind Varianten der Erfindung gezeigt, bei der jeweils zwei Elastomerelemente 71, 710, 72, 720 zwischen den Abstützflächen 610, 620 und dem Blattfederelement 40 angeordnet sind. Die jeweiligen Elastomerelemente 71, 710, 72, 720 können auf unterschiedliche Arten und Weisen an der jeweiligen Komponente, also der Lagerungskomponente 600 und dem Blattfederelement 40, befestigt sein. In einem Ausführungsbeispiel ist es vorgesehen, dass das auf der Lagerungskomponente 600 an der Anlagefläche angeordnete Elastomerelement 710, 720 formschlüssig an der Lagerungskomponente 600 befestigt ist, während das andere Elastomerelement 71, 72 auf der Oberfläche des Blattfederelementes 40 verklebt ist. Das obere Elastomerelement 710, 720 kann Seitenwände oder eine Ausnehmung aufweisen, in die das untere Elastomerelement 71, 72 eingeführt bzw. daran angelegt werden kann, sodass eine Verschiebung und/oder Verdrehung der Elastomerelemente 71, 710, 72, 720 zueinander nicht oder nur in bestimmten Richtungen möglich ist. Je nach Formgebung der Elastomerelemente kann eine Verdrehung um eine Achse senkrecht zu der Transversalebene möglich sein. Eine Verdrehung und Verkippung der Lagerungskomponente 600 relativ zu dem Blattfederelement 40 ist aufgrund der elastischen Eigenschaften der Elastomerelemente 71, 710, 72, 720 weiterhin möglich. Es besteht ebenfalls die Möglichkeit, dass unterschiedliche Befestigungsarten an unterschiedlichen Positionen angewendet werden, sodass beispielsweise das obere Elastomerelement 710 in dem vorderen Bereich der Lagerungskomponente 600 verklebt und das untere Elastomerelement 71 formschlüssig befestigt oder über eine Klemmeinrichtung an dem Blattfederelement 40 festgelegt ist. Grundsätzlich können auch beide Elastomerelemente 71, 710 mit der jeweiligen Komponente verklebt sein. Die Anordnung mehrerer Elastomerelemente 71, 710; 72, 720 zwischen der Lagerungskomponente 600 und dem Blattfederelement 40 vergrößert die Gestaltungsfreiheit bei der Auslegung der elastischen Eigenschaften. Bei einer auswechselbaren Befestigung eines Elastomerelementes können vereinfacht Anpassungen an die jeweiligen Vorlieben des jeweiligen Nutzers vorgenommen werden. Möchte ein Nutzer beispielsweise eine geringere Nachgiebigkeit an der einen oder anderen Stelle des orthopädietechnischen Systems bzw. des Prothesenfußes haben, kann dies durch den Austausch beispielsweise der formschlüssig über eine Verschraubung an der Lagerungskomponente 600 befestigten Elastomerelemente 710, 720 erfolgen. Aufgrund der bevorzugt metallischen Ausgestaltung der Lagerungskomponente 600 ist eine Verschraubung von Elastomerelementen 710, 720 an der Lagerungskomponente 600 leicht möglich, ohne dass eine nennenswerte strukturelle Beeinträchtigung der Festigkeitseigenschaften oder mechanischen Eigenschaften auftritt.

Um zu verhindern, dass bei nicht einander fixierten Elastomerelementen 71, 710; 72, 720 eine Trennung zwischen den Elastomerelementen 71, 710; 72, 720 stattfindet, sind in dem dargestellten Ausführungsbeispiel Vorspanneinrichtungen 60 in Gestalt von Gurten sowohl an der Lagerungskomponente 600 als auch an dem Blattfederelement 40 angeordnet. Die Vorspanneinrichtungen 60 können flexibel und zugstarr oder elastisch ausgebildet sein. Sie sind in Führungen an der Lagerungskomponente 600 und unterhalb der Blattfederelementes 40 herumgeführt und bewirken eine Vorspannung der Lagerungskomponente 600 in Richtung auf das Blattfederelement 40. Dadurch werden die paarweise angeordneten Elastomerelemente 71, 710, 72, 720 aufeinandergepresst und dauerhaft aneinandergehalten. Die Vorspanneinrichtung 60 kann auch lösbar ausgebildet sein, sodass zum Auswechseln von Elastomerelementen 71, 710, 72, 720 die Vorspanneinrichtung 60 jeweils gelöst und wieder angelegt wird. Die Vorspanneinrichtung 60 kann auch anders ausgebildet sein, beispielsweise als eine Verschraubung.

In der Figur 8 ist zu erkennen, dass die seitlichen Anschläge 630 beanstandet sowohl zu dem Blattfederelement 40 als auch zu dem unteren, an dem Blattfederelement 40 befestigten Elastomerelement 71 positioniert sind. Das obere, an der Lagerungskomponente 600 angeordnete Elastomerelement 710 füllt den Abstand oder Freiraum zwischen der Innenseite des Anschlages 630 und dem unteren Elastomerelement 71 aus, sodass eine zusätzliche seitliche Führung bereitgestellt wird. Neben einer Überbrückung dieses Abstandes können die Seitenwände des oberen Elastomerelementes 710 auch einen Freiraum zu den inneren Oberflächen des jeweiligen Anschlages 630 oder nur eines Anschlages 630 freilassen. Je nach Ausgestaltung der Elastomerelemente ist eine Beeinflussung des Bewegungsverhaltens der Lagerungskomponente 600 relativ zu dem Blattfederelement 40 möglich.

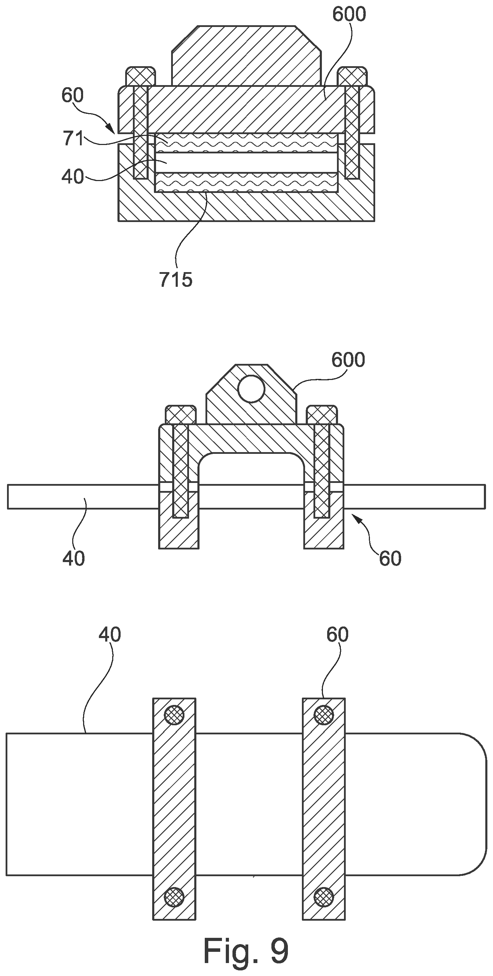

In der Figur 9 sind drei Darstellungen einer weiteren Variante gezeigt, bei der das Blattfederelement 40 zwischen zwei Elastomerelementen 71, 715 eingebettet ist. Ein Elastomerelement 71 ist zwischen der Lagerungskomponente 600 an der Abstützfläche und der Oberseite des Blattfederelementes 40 angeordnet, das andere Elastomerelement 715 ist unterhalb des Blattfederelementes 40 angeordnet und stützt dieses gegenüber einer Vorspanneinrichtung 60 ab, die in dem dargestellten Ausführungsbeispiel als ein Klemmbügel ausgebildet ist, der über zwei Schrauben an der Lagerungskomponente 600 lösbar fixiert ist. Der Klemmbügel erstreckt sich unterhalb des Blattfederelementes 40 und ist U-förmig ausgebildet. In den beiden nach oben ragenden Schenkeln des Klemmbügels sind Innengewinde zur Aufnahme der Schrauben angeordnet. Das Blattfederelement 40 wird seitlich durch die beiden nach oben ragenden Schenkel geführt, wobei zwischen den Innenseiten der nach oben ragenden Schenkel und dem Blattfederelement 40 ein seitlicher Abstand bestehen kann, um eine Verdrehung sowie Verschiebung relativ zu der Lagerungskomponente 600 zu ermöglichen. Die beiden Schrauben sind durch Durchgangslöcher in der Lagerungskomponente 600 geführt, durch Lösen oder Anspannen der Schrauben lässt sich die Vorspannung der Elastomerkomponenten 71, 715 individuell variieren. In der unteren Darstellung der Figur 9 ist eine Untenansicht des orthopädietechnischen Systems als Teil eines Prothesenfußes gezeigt, aus der zu erkennen ist, dass der jeweilige Klemmbügel sich über die gesamte Breite unterhalb des Blattfederelementes 40 erstreckt. Auch hier sind zwei in Längserstreckung des Blattfederelementes 40 zueinander beabstandete Vorspanneinrichtungen 60 an der Lagerungskomponente 600 angeordnet und ermöglichen eine Krafteinleitung in zwei voneinander beabstandeten Bereichen des Blattfederelementes 40. Die Vorspannung lässt sich stufenlos einstellen. Neben einer U-förmigen Ausgestaltung des Klemmbügels kann die Vorspanneinrichtung 60 auch lediglich als eine streifenförmige Platte ausgebildet sein. Statt einer Schraube können auch andere Kraftübertragungseinrichtungen Teil der Vorspanneinrichtung 60 sein, um das Blattfederelement 40 gegenüber der Lagerungskomponente 600 mit zumindest einem dazwischen angeordneten Elastomerelement 71 gegeneinander vorzuspannen und aneinander festzuklemmen.

Das Blattfederelement stützt sich vorteilhafterweise an seinen beiden Endbereichen auf anderen Komponenten oder dem Fußboden ab, sodass insgesamt eine Vierpunkt-Lagerung mit einer Lagerung an den beiden Endbereichen sowie einer Krafteinleitung an zwei voneinander beabstandeten, zwischen den Lagerungsstellen an den Endbereichen vorgesehenen Krafteinleitungsbereichen erfolgt. Bei einer Ausgestaltung der Lagerungskomponente 600 als Lagerbock mit einer Lageraufnahme 640 zwischen zwei Abstützflächen 610, 620, ist eine Positionierung der Lageaufnahme 640 auf dem Blattfederelement 40 in der Mitte der Federlänge oder innerhalb des mittleren Fünftels des Blattfederelementes 40 vorteilhaft. Dadurch wird eine gleichmäßige Belastung des Blattfederelementes 40 gewährleistet. Bezogen auf die Gesamtlänge des Prothesenfußeinsatzes ist aufgrund der größeren Gesamtlänge der Basisfeder 20 im Vergleich zu dem Blattfederelement 40 und der nicht mittigen Anordnung des Blattfederelementes 40 über der Basisfeder 20 die Positionierung der Lageraufnahme 640 etwas weiter in anteriorer Richtung verschoben. In einem vollständig montierten Prothesenfuß mit einer Kosmetik befindet sich die Lageraufnahme 640 im Vergleich zu der Gesamtlänge etwas hinter der Mitte, jedoch immer noch innerhalb des mittleren Fünftels der Gesamtlänge des Prothesenfußes.

### Bevorzugte Merkmale der Anmeldung

1. Orthopädietechnisches System mit einem Blattfederelement (40) und einer auf der Hauptfläche des Blattfederelements (40) abgestützten Lagerungskomponente (600), die an dem Blattfederelement (40) festgelegt ist, dadurch gekennzeichnet, dass zwischen der Lagerungskomponente (600) und der Hauptfläche (41) des Blattfederelementes (40) zumindest ein Elastomerelement (71, 72) angeordnet ist, das Zugkräfte und Scherkräfte übertragend an dem Blattfederelement (40) und der Lagerungskomponente (600) befestigt ist.
2. Orthopädietechnisches System nach Absatz 1, dadurch gekennzeichnet, dass die Lagerungskomponente (600) zumindest eine ebene Abstützfläche (610; 620) aufweist.
3. Orthopädietechnisches System nach Absatz 1 oder 2, dadurch gekennzeichnet, dass die Lagerungskomponente (600) mehrere voneinander beabstandete Abstützflächen (610, 620) aufweist.
4. Orthopädietechnisches System nach einem der Absätze 1 bis 3, dadurch gekennzeichnet, dass Elastomerelement (71, 72) über eine Vorspanneinrichtung (60) an dem Blattfederelement (40) und/oder der Lagerungskomponente (600) befestigt ist.
5. Orthopädietechnisches System nach einem der Absätze 1 bis 4, dadurch gekennzeichnet, dass das Elastomerelement (71, 72) mit der Lagerungskomponente (600) und/oder dem Blattfederelement (40) verklebt ist.
6. Orthopädietechnisches System nach einem der Absätze 1 bis 5, dadurch gekennzeichnet, dass an der Lagerungskomponente (600) zumindest ein Anschlag (630) angeordnet ist, der in einer Ausgangsstellung beabstandet zu einer Nebenfläche (43) und/oder einem Anschlagelement des Blattfederelementes (40) angeordnet ist.
7. Orthopädietechnisches System nach Absatz 6, dadurch gekennzeichnet, dass mehrere Anschläge einander gegenüberliegend angeordnet sind.
8. Orthopädietechnisches System nach einem der Absätze 1 bis 7, dadurch gekennzeichnet, dass die Lagerungskomponente (600) in drei rotatorischen und translatorischen Freiheitsgraden elastisch auf dem Blattfederelement (40) gelagert ist.
9. Orthopädietechnisches System nach einem der Absätze 1 bis 8, dadurch gekennzeichnet, dass das Blattfederelement (40) aus einem Faserverbundwerkstoff und die Lagerungskomponente (600) aus einem Metall hergestellt sind.
10. Orthopädietechnisches System nach einem der Absätze 1 bis 9, dadurch gekennzeichnet, dass die Lagerungskomponente (600) als ein Lagerbock mit einer Lageraufnahme (640) ausgebildet ist.
11. Orthopädietechnisches System nach einem der Absätze 1 bis 9, dadurch gekennzeichnet, dass an der Lagerungskomponente (600) eine weitere Komponente (10) drehstarr oder elastisch befestigt oder ausgebildet ist.
12. Orthopädietechnisches System nach einem der Absätze 1 bis 11, dadurch gekennzeichnet, dass die Lagerungskomponente (600) in dem mittleren Drittel zwischen den Enden des Blattfederelementes (40) angeordnet ist.
13. Prothesenfußeinsatz mit einem orthopädietechnischen System nach einem der Absätze 1 bis 12.

## Patentansprüche

1. Orthopädietechnisches System mit einem Blattfederelement (40) und einer auf der Hauptfläche des Blattfederelements (40) abgestützten Lagerungskomponente (600), die an dem Blattfederelement (40) festgelegt ist, wobei zwischen der Lagerungskomponente (600) und der Hauptfläche (41) des Blattfederelementes (40) zumindest ein Elastomerelement (71, 72) angeordnet ist, das Zugkräfte und Scherkräfte übertragend an dem Blattfederelement (40) und der Lagerungskomponente (600) befestigt ist, **dadurch gekennzeichnet, dass** an der Lagerungskomponente (600) zumindest ein Anschlag (630) angeordnet ist, der in einer Ausgangsstellung beabstandet zu einer Nebenfläche (43) und/oder einem Anschlagelement des Blattfederelementes (40) angeordnet ist.

2. Orthopädietechnisches System nach Anspruch 1, **dadurch gekennzeichnet, dass** die Lagerungskomponente (600) zumindest eine ebene Abstützfläche (610; 620) aufweist.

3. Orthopädietechnisches System nach Anspruch 1 oder 2, **dadurch gekennzeichnet, dass** die Lagerungskomponente (600) mehrere voneinander beabstandete Abstützflächen (610, 620) aufweist.

4. Orthopädietechnisches System nach einem der voranstehenden Ansprüche, **dadurch gekennzeichnet, dass** Elastomerelement (71, 72) über eine Vorspanneinrichtung (60) an dem Blattfederelement (40) und/oder der Lagerungskomponente (600) befestigt ist.

5. Orthopädietechnisches System nach einem der voranstehenden Ansprüche, **dadurch gekennzeichnet, dass** das Elastomerelement (71, 72) mit der Lagerungskomponente (600) und/oder dem Blattfederelement (40) verklebt ist.

6. Orthopädietechnisches System nach Anspruch 1, **dadurch gekennzeichnet, dass** mehrere Anschläge einander gegenüberliegend angeordnet sind.

7. Orthopädietechnisches System nach einem der voranstehenden Ansprüche, **dadurch gekennzeichnet, dass** die Lagerungskomponente (600) in drei rotatorischen und translatorischen Freiheitsgraden elastisch auf dem Blattfederelement (40) gelagert ist.

8. Orthopädietechnisches System nach einem der voranstehenden Ansprüche, **dadurch gekennzeichnet, dass** das Blattfederelement (40) aus einem Faserverbundwerkstoff und die Lagerungskomponente (600) aus einem Metall hergestellt sind.

9. Orthopädietechnisches System nach einem der voranstehenden Ansprüche, **dadurch gekennzeichnet, dass** die Lagerungskomponente (600) als ein Lagerbock mit einer Lageraufnahme (640) ausgebildet ist.

10. Orthopädietechnisches System nach einem der Ansprüche 1 bis 8, **dadurch gekennzeichnet, dass** an der Lagerungskomponente (600) eine weitere Komponente (10) drehstarr oder elastisch befestigt oder ausgebildet ist.

11. Orthopädietechnisches System nach einem der voranstehenden Ansprüche, **dadurch gekennzeichnet, dass** die Lagerungskomponente (600) in dem mittleren Drittel zwischen den Enden des Blattfederelementes (40) angeordnet ist.

12. Prothesenfußeinsatz mit einem orthopädietechnischen System nach einem der voranstehenden Ansprüche.
